# EUROPEAN PATENT APPLICATION

(11) **EP 4 591 790 A1**
(43) Date of publication of application: **30.07.2025**
(21) Application number: 23881524.5
(22) Date of filing: 19.09.2023
(51) Int. Cl.: A61B 5/145

(54) **IMPLANTABLE APPARATUS**

(30) Priority: 24.10.2022 CN 202222801795 U
(71) Applicant: Huawei Technologies Co., Ltd., Shenzhen, Guangdong 518129 (CN)
(72) Inventor: ZOU, Lin, Shenzhen, Guangdong 518129 (CN); JIN, Junye, Shenzhen, Guangdong 518129 (CN); ZHAO, Menglong, Shenzhen, Guangdong 518129 (CN)
(74) Representative: Thun, Clemens
(86) International application number: PCT/CN2023/119606
(87) International publication number: WO 2024/087944

(57) **Abstract**

An implantation apparatus includes a first housing (1), a second housing (2), and a door plate assembly (3). An implantation assembly is disposed in the first housing (1). The second housing (2) movably penetrates the first housing (1). A through hole (21) for the implantation assembly to pass through is provided on the first housing (1). The door plate assembly (3) is movably connected to the second housing (2), and is configured to seal or open the through hole (21). The movable door plate assembly (3) is used, so that the implantation assembly cannot be seen before a user presses the second housing (2) or in a housing pressing process. This can avoid a psychological burden of the user for a structure like a hard needle.

## Description

The present invention claims priority to Chinese Patent Application No. 202222801795.9, filed with the China National Intellectual Property Administration on October 24, 2022 and entitled "IMPLANTATION APPARATUS", which is incorporated herein by reference in its entirety.

### TECHNICAL FIELD

This application relates to the field of sensor implantation technologies, and in particular, to an implantation apparatus.

### BACKGROUND

Continuous glucose monitoring (Continuous Glucose Monitoring, CGM) is to monitor glucose concentrations in a subcutaneous tissue via a glucose sensor, to indirectly reflect blood glucose levels. A hard needle for implanting a sensor exists in an existing CGM measurement instrument. In an implantation process, the hard needle is exposed in a visual field, that is, can be clearly seen by a user, increasing a psychological burden of the user.

### SUMMARY

An objective of this application is to provide an implantation apparatus, to resolve the foregoing conventional-technology problem that exposure of a hard needle of a CGM measurement instrument in a visual field in an implantation operation process increases a psychological burden of a user.

This application provides an implantation apparatus, including:
a first housing, where an implantation assembly is disposed in the first housing;
a second housing, where the second housing movably penetrates the first housing, and a through hole for the implantation assembly to pass through is provided on the first housing; and
a door plate assembly, movably connected to the second housing, and configured to seal or open the through hole.

According to the implantation apparatus provided in this application, the movable door plate assembly is used, so that the implantation assembly cannot be seen before a user presses the second housing or in a housing pressing process. This can avoid a psychological burden of the user for a structure like a hard needle.

In a possible design, the door plate assembly includes a door plate and a first elastic member. One end of the first elastic member is connected to the second housing, and the other end of the first elastic member is connected to the door plate. The first elastic member is in a compressed state when the door plate is in a state of sealing the through hole.

When the second housing is pressed, the second housing moves relative to the first housing. This can cancel the state of sealing the through hole by the door plate. In a process of restoring deformation, the first elastic member releases elastic potential energy, and can pull the door plate to move relative to the second housing, so that the door plate cancels shielding for the through hole. In this way, the door plate assembly is automatically opened when the second housing is pressed.

In a possible design, two door plates are symmetrically disposed. Each door plate is connected to the second housing through at least one first elastic member. The two door plates move reversely through the first elastic members respectively.

When the through hole is in a sealed state, the two door plates butt each other, and a butting position may be located in the symmetric center of the through hole. In this way, the two door plates can have a same movement stroke and same movement duration when moving and being opened. Specifically, when the two door plates are opened, the first elastic members correspondingly connected to the two door plates may make the two door plates move reversely. In this way, the two door plates both move half of a size of the through hole, and the through hole can be completely opened. After the door plates are completely opened, the implantation assembly does not reach the through hole yet. This can avoid interference between the implantation assembly and the door plates.

In a possible design, each door plate is connected to the second housing through two first elastic members. In a movement direction perpendicular to the door plate, two first elastic members connected to one door plate are symmetrically distributed.

Each door plate is connected to the two first elastic members, so that each door plate can obtain greater driving force, to improve a speed of opening the door plate. In addition, the two first elastic members connected to each door plate are symmetrically distributed, so that even driving force can be applied to the door plate. This ensures movement stability of the door plate and facilitates quick opening of the door plate.

In a possible design, the door plate assembly further includes a guide structure. The guide structure is connected to the second housing. The door plate is slidably connected to the guide structure. The door plate is slidably connected to the guide structure, to ensure movement stability of the door plate, and facilitate quick opening of the door plate.

In a possible design, the guide structure is a guide rail.

In a possible design, the implantation apparatus further includes a limiting assembly. The limiting assembly is connected to the second housing, and is configured to limit the door plate assembly to keep the sealed state of the through hole. When the door plate assembly seals the through hole, the limiting assembly can cooperate with the door plate assembly, to prevent the door plate assembly from being opened. In this way, when the implantation apparatus is not used, the through hole can be kept sealed through cooperation between the door plate assembly and the limiting assembly.

In a possible design, the limiting assembly includes a lever and a second elastic member. The lever includes a first end and a second end. A rotating part is disposed in a position that is on the lever and that is located between the first end and the second end. The rotating part is rotatably connected to the second housing. One end of the second elastic member is connected to the second housing, and the other end of the second elastic member is connected to the first end of the lever. An end that is of the first end of the lever and that is away from the second elastic member is configured to limit the door plate assembly to keep the sealed state of the through hole. A first protrusion is disposed on an inner side wall of the first housing. At least a part of the second end of the lever blocks the first protrusion.

When the second housing is pressed, the second housing moves downward relative to the first housing, and the lever moves downward synchronously with the second housing. Because the at least part of the second end blocks the first protrusion, when passing through the first protrusion, the second end is pushed toward the inside of the first housing by the first protrusion, so that the rotating part of the lever rotates relative to the second housing, and the first end of the lever moves in a direction away from the door plate assembly. In this way, the first end can be separated from the door plate assembly, and after the door plate assembly is not constrained by the lever, the door plate can be automatically opened under an action of the first elastic member. A surface that is of the first protrusion and that faces the second housing can be an oblique surface. In this way, when moving downward, the lever can slide through guide of the oblique surface of the first protrusion to pass through the first protrusion, to avoid sticking of the lever at the first protrusion.

In a possible design, a clamping jaw is disposed at the first end of the lever. A clamping slot is provided on the door plate assembly. The clamping jaw is in clamping cooperation with the clamping slot. When the door plate assembly seals the through hole, the clamping jaw of the lever can be clamped into the clamping slot. This can prevent the door plate assembly from being opened. When the second housing is pressed down, the lever can rotate to separate the clamping jaw from the clamping slot, and the door plate assembly that is not constrained by the clamping jaw can be automatically opened.

In a possible design, the limiting assembly includes a button and a third elastic member. The button is slidably connected to the second housing. The button partially protrudes from the upper surface of the second housing. A limiting part is disposed on the button. The limiting part is configured to limit the door plate assembly to keep the sealed state of the through hole. One end of the third elastic member is connected to the button, and the other end of the third elastic member is connected to the second housing.

When the user presses the second housing, because the button protrudes from the upper surface of the second housing, the user preferably presses the button. The button moves downward prior to the second housing, so that the limiting part of the button is separated from the door plate assembly. After the door plate assembly is not constrained by the limiting part, the door plate assembly can be automatically opened. When the door plate assembly is not constrained by the limiting part, a skin of the user can be in contact with the upper surface of the second housing and cover the through hole. In this way, in a process of opening the door plate assembly, because the through hole is shielded by the skin, the user cannot see the internal implantation assembly through the through hole, reducing the psychological burden of the user.

In a possible design, the limiting part includes a clamping jaw. A clamping slot is provided on the door plate assembly. The clamping jaw is in clamping cooperation with the clamping slot. When the door plate assembly seals the through hole, the clamping jaw can be clamped into the clamping slot. This can prevent the door plate assembly from being opened. When the button is pressed down, the clamping jaw can be separated from the clamping slot, and the door plate assembly that is not constrained by the clamping jaw can be automatically opened.

In a possible design, the limiting assembly further includes a support member. The support member is fastened to the second housing. Activity space for the button to move is disposed between the second housing and a position that is of the support member and that is opposite to the button. An end that is of the third elastic member and that is away from the button is connected to the support member.

The support member can ensure reliable support for the button and the third elastic member. The activity space is disposed between the support member and the second housing, so that the button can move to the second housing after being pressed. In this way, the limiting part of the button can be separated from the door plate assembly, a constraint on the door plate assembly is canceled, and the door plate assembly can be automatically opened.

In a possible design, a support part is disposed at the bottom of the second housing. A second protrusion is disposed on an inner wall of the first housing. In a movement direction of the second housing, at least a part of the support part laps the second protrusion.

When the implantation apparatus is not used, the second housing can be supported on the second protrusion of the first housing via the support part, to prevent the second housing from falling into the first housing. When the second housing is pressed under appropriate external force, the support part can undergo appropriate elastic deformation after being stressed. In one aspect, the support part can absorb the force applied to the second housing, to buffer a movement of the second housing, so as to prevent bringing discomfort to the user when the second housing falls down quickly when being greatly stressed. In another aspect, the support part can deviate through elastic deformation, to cancel a lapping state with the second protrusion, so that the support part can pass through the second protrusion with the movement of the second housing, the second housing can smoothly fall into the first housing, and the implantation assembly can complete an implantation action after extending out from the through hole.

It should be understood that the foregoing general descriptions and the following detailed descriptions are merely examples, and are not intended to limit this application.

### BRIEF DESCRIPTION OF DRAWINGS

FIG. 1 is a diagram of a structure of an implantation apparatus (a door plate assembly is in a closed state) according to an embodiment of this application;
FIG. 2 is a diagram of a structure of an implantation apparatus (a door plate assembly is in an opened state) according to an embodiment of this application;
FIG. 3 is a diagram of a structure of an implantation apparatus (an implantation assembly is in an extending state) according to an embodiment of this application;
FIG. 4 is a diagram of a structure of an implantation apparatus (a state after an implantation assembly is transferred to a skin) according to an embodiment of this application;
FIG. 5 is a state diagram when a door plate is closed according to an embodiment of this application;
FIG. 6 is a state diagram when a door plate is opened according to an embodiment of this application;
FIG. 7 is a state diagram when a door plate is closed according to another embodiment of this application;
FIG. 8 is a state diagram when a door plate is opened according to another embodiment of this application;
FIG. 9 is a sectional view of an implantation apparatus in a position A-A in FIG. 1 according to an embodiment of this application;
FIG. 10 is an enlarged view of a position C in FIG. 9;
FIG. 11 is a diagram of a structure of a lever;
FIG. 12 is a sectional view of an implantation apparatus in a position A-A in FIG. 1 according to another embodiment of this application;
FIG. 13 is a diagram of a structure in which a limiting assembly is disposed on an inner side of a cover plate;
FIG. 14 is a top view in which a limiting assembly cooperates with a door plate;
FIG. 15 is a state diagram when a limiting assembly is locked with a door plate;
FIG. 16 is a state diagram when a limiting assembly is unlocked with a door plate;
FIG. 17 is a state diagram when a door plate is closed according to still another embodiment of this application;
FIG. 18 is a state diagram when a door plate is opened according to still another embodiment of this application; and
FIG. 19 is a sectional view of an implantation apparatus in a position B-B in FIG. 1 according to an embodiment of this application.

### Reference numerals:

1: first housing;
   11: first protrusion;
   12: second protrusion;
2: second housing;
   21: through hole;
   22: connection block;
   23: support part;
   24: cover plate;
3: door plate assembly;
   31: door plate;
   32: first elastic member;
   33: guide structure;
   34: clamping slot;
4: limiting assembly;
   41: lever;
      411: first end;
      412: second end;
      413: rotating part;
   42: second elastic member;
   43: clamping claw;
   44: button;
      441: limiting part;
   45: third elastic member;
   46: support member;
   47: activity space;
   48: screw;
5: implantation assembly; and
   51: hard needle.

The accompanying drawings herein are incorporated into this specification and constitute a part of this specification, show embodiments in accordance with this application, and are used together with this specification to explain the principle of this application.

### DESCRIPTION OF EMBODIMENTS

To make the objectives, technical solutions, and advantages of this application clearer and more comprehensible, the following further describes this application in detail with reference to the accompanying drawings and embodiments. It should be understood that specific embodiments described herein are used merely to explain this application but are not intended to limit this application.

In descriptions of this application, unless otherwise specified and limited, the terms "first" and "second" are merely intended for a purpose of description, and should not be understood as an indication or implication of relative importance. Unless otherwise specified or stated, the term "a plurality of" means two or more than two. The terms "connection", "fastening", and the like should be understood in a broad sense. For example, the "connection" may be a fixed connection, or may be a detachable connection, an integrated connection, or an electrical connection, or may be a direct connection, or may be an indirect connection through an intermediate medium. A person of ordinary skill in the art may understand specific meanings of the foregoing terms in this application based on a specific case.

In the descriptions of this specification, it should be understood that the orientation words such as "upper" and "lower" described in embodiments of this application are described from perspectives shown in the accompanying drawings, and should not be construed as a limitation on embodiments of this application. In addition, in the context, it should be further understood that when it is mentioned that one element is connected "above" or "below" another element, the element can be directly connected "above" or "below" the another element, or may be indirectly connected "above" or "below" the another element through an intermediate element.

Continuous glucose monitoring (Continuous Glucose Monitoring, CGM) is to monitor glucose concentrations in a subcutaneous tissue via a glucose sensor, to indirectly reflect blood glucose levels. A hard needle for implanting a sensor exists in an existing CGM measurement instrument. When the hard needle is not used, a cover of the CGM measurement instrument can shield the hard needle inside the measurement instrument, to prevent a dusty impurity and the like from entering the measurement instrument. When the hard needle is used, the cover needs to be removed, so that the hard needle is exposed, to help implant the sensor into a skin through assistance of the hard needle. In an implantation process, the hard needle is always exposed in a visual field, that is, can be clearly seen by a user, increasing a psychological burden of the user.

This embodiment provides an implantation apparatus. The implantation apparatus may be used in a product that requires auxiliary implantation, for example, a CGM measurement instrument. For ease of description, in this embodiment, an example in which the implantation apparatus is used in the CGM measurement instrument is used for description.

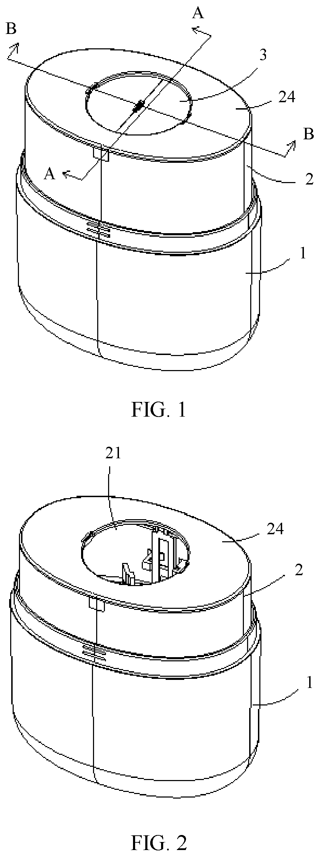

FIG. 1 is a diagram of a structure of an implantation apparatus (a door plate assembly is in a closed state) according to an embodiment of this application. FIG. 2 is a diagram of the structure of the implantation apparatus (the door plate assembly is in an opened state) according to an embodiment of this application. As shown in FIG. 1 and FIG. 2, the implantation apparatus includes a first housing 1, a second housing 2, and the door plate assembly 3. An implantation assembly is disposed in the first housing 1. The second housing 2 movably penetrates the first housing 1. A through hole 21 for the implantation assembly to pass through is provided on the first housing 1. The door plate assembly 3 is movably connected to the second housing 2, and is configured to seal or open the through hole 21.

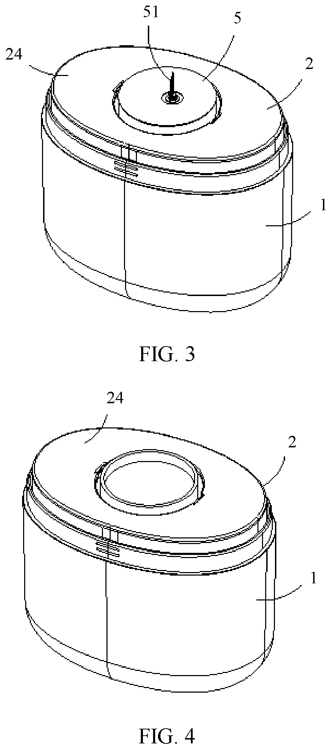

The implantation assembly may include a hard needle, a blood glucose transmitter, a blood glucose sensor, and the like. The blood glucose sensor is soft, and the blood glucose sensor is difficult to be independently implanted into a skin. Therefore, a hard needle with high hardness is required to assist in implanting the blood glucose sensor into the skin. For example, as shown in FIG. 1 and FIG. 2, when implantation needs to be performed on a skin of an arm, a position on which implantation needs to be performed on the arm may be aligned with the through hole 21 on the second housing 2, and then the second housing 2 is continuously pressed, so that the second housing 2 moves toward the inside of the first housing 1. In an implantation process, the implantation assembly in the first housing 1 keeps static relative to the first housing 1. In other words, when the second housing 2 moves toward the inside of the first housing 1, the second housing 2 and the implantation assembly move relative to each other. To be specific, after the second housing 2 moves to a specific stroke, as shown in FIG. 3, the implantation assembly may pass through the through hole 21, so that the hard needle is punctured into the skin of the arm, to implement implantation of the blood glucose sensor. In addition, after the blood glucose transmitter is in contact with the skin, the blood glucose transmitter may be pasted on the skin via double-sided adhesive tape on a surface of the transmitter, as shown in FIG. 4. After the second housing 2 moves to an end, a needle retraction mechanism in the first housing 1 may be triggered, and the needle retraction mechanism may pull out the hard needle from a human body, to complete all implantation operations.

If the through hole 21 is in an opened state before the arm presses the second housing 2, the implantation assembly is exposed in a visual field of a user through the through hole 21, that is, the user can clearly see the hard needle. This causes a great psychological burden to the user. Therefore, as shown in FIG. 1, in this embodiment, the door plate assembly 3 is disposed at the through hole 21, and the door plate assembly 3 is movably connected to the second housing 2. To be specific, when the user does not press the second housing 2, through a movement relative to the second housing 2, the door plate assembly 3 may make the through hole 21 keep a sealed state, and before pressing the second housing 2, the user is prevented from seeing the implantation assembly. As shown in FIG. 2, when the user presses the second housing 2, the door plate assembly 3 may be automatically opened, so that the implantation assembly can pass through the through hole 21. In this case, because the second housing 2 needs to be kept pressed, the position on which implantation needs to be performed, for example, the arm of the user, covers the through hole 21, and the through hole 21 and the internal implantation assembly are not seen. In this way, the movable door plate assembly 3 is used, so that the implantation assembly cannot be seen before the user presses the second housing 2 or in a housing pressing process. This can avoid a psychological burden of the user for a structure like the hard needle.

The first housing 1 and the second housing 2 may be tubular structures, for example, cylindrical tubular structures, elliptical cylindrical tubular structures, or prismatic tubular structures. A height of the first housing 1 is greater than a height of the second housing 2, so that the second housing 2 can move to the first housing 1, to help the implantation assembly in the first housing 1 pass through the through hole 21 of the second housing 2. As shown in FIG. 2, a cover plate 24 is disposed at the top of the second housing 2, and the through hole 21 may be provided on the cover plate 24. In the implantation process, a skin tissue of the user can press the cover plate 24 of the second housing 2, so that the cover plate 24 can make a large contact area exist between the skin of the user and the second housing 2. This improves comfort of pressing the second housing 2 by the user, and can ensure stability of the second housing 2 when the second housing 2 is pressed to move.

Specifically, the door plate assembly 3 includes a door plate 31 and a first elastic member 32. One end of the first elastic member 32 is connected to the second housing 2, and the other end of the first elastic member 32 is connected to the door plate 31. The first elastic member 32 is in a compressed state when the door plate 31 is in a state of sealing the through hole 21.

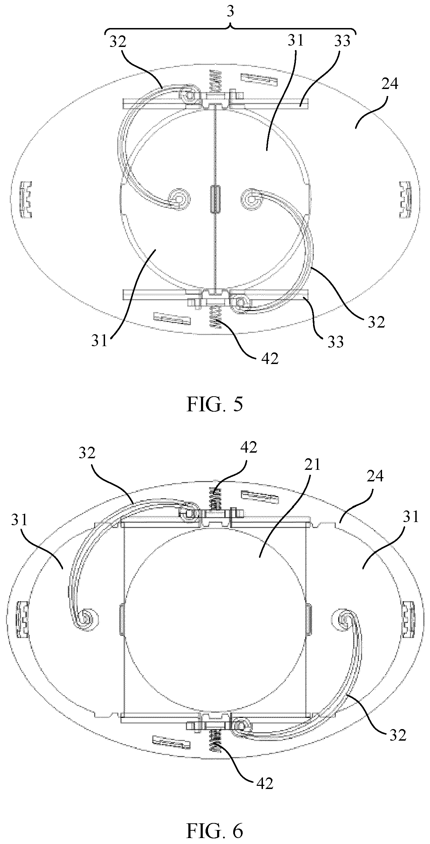

As shown in FIG. 1 and FIG. 5, the door plate 31 may be a flat-plate-like structure. When the implantation apparatus is not used, the door plate 31 can seal the through hole 21, and the implantation assembly inside the implantation apparatus cannot be seen through the through hole 21. In this case, the first elastic member 32 is in the compressed state. As shown in FIG. 2 and FIG. 6, when the second housing 2 is pressed in use, the second housing 2 moves relative to the first housing 1. This can cancel the state of sealing the through hole 21 by the door plate 31. In a process of restoring deformation, the first elastic member 32 releases elastic potential energy, and can pull the door plate 31 to move relative to the second housing 2, so that the door plate cancels shielding for the through hole 21. In this way, the door plate assembly 3 is automatically opened when the second housing 2 is pressed. In this way, through cooperation between the door plate 31 and the first elastic member 32, the door plate 31 can be automatically opened when the second housing 2 is pressed. In other words, when the skin seals the through hole 21 and presses the second housing 2, the user does not see the implantation assembly. In addition, after the door plate 31 is automatically opened, the implantation assembly can pass through the through hole 21, and interference is not caused to the implantation assembly. The user needs only to press the second housing 2 to complete the implantation operation. It is convenient to use the implantation apparatus, and the user can also be prevented from having the psychological burden when seeing the implantation assembly.

The door plate 31 may be disposed at a lower part of the cover plate 24 of the second housing 2, namely, a side that is of the cover plate 24 and that faces the first housing 1. In this way, an appearance of the implantation apparatus can be clean and concise, and movable parts such as the door plate 31 and the first elastic member 32 can be protected from collision or contamination. In addition, to facilitate assembling of the first elastic member 32, the first elastic member 32 may be a spring, a dome, or the like.

Specifically, one door plate 31 may be disposed, and one first elastic member 32 may also be correspondingly disposed. In this way, fewer parts are used, and the structure is simple. However, the door plate 31 needs to move a large stroke when changing from a closed state to a fully opened state, and the first elastic member 32 also requires great force to ensure that the door plate 31 is pulled from one end to the other end of the through hole 21. Consequently, it takes a long time to completely open the door plate 31, and interference between the implantation assembly and the door plate 31 caused when the door plate 31 is not completely opened but the implantation assembly reaches the through hole 21 is likely to occur.

In this embodiment, as shown in FIG. 5 and FIG. 6, two door plates 31 are symmetrically disposed. Each door plate 31 is connected to the second housing 2 through at least one first elastic member 32. The two door plates 31 move reversely through the corresponding first elastic members 32 respectively. In other words, when the through hole 21 is in the sealed state, the two door plates 31 butt each other, and a butting position may be located in the symmetric center of the through hole 21. In this way, the two door plates 31 can have a same movement stroke and same movement duration when moving and being opened. Specifically, when the two door plates 31 are opened, the first elastic members 32 correspondingly connected to the two door plates 31 may make the two door plates 31 move reversely. In this way, the two door plates 31 both move half of a size of the through hole 21, and the through hole 21 can be completely opened. After the door plates 31 are completely opened, the implantation assembly does not reach the through hole 21 yet. This can avoid interference between the implantation assembly and the door plates 31.

Specifically, each door plate 31 is connected to the second housing 2 through two first elastic members 32. In a movement direction perpendicular to the door plate 31, two first elastic members 32 connected to one door plate 31 are symmetrically distributed.

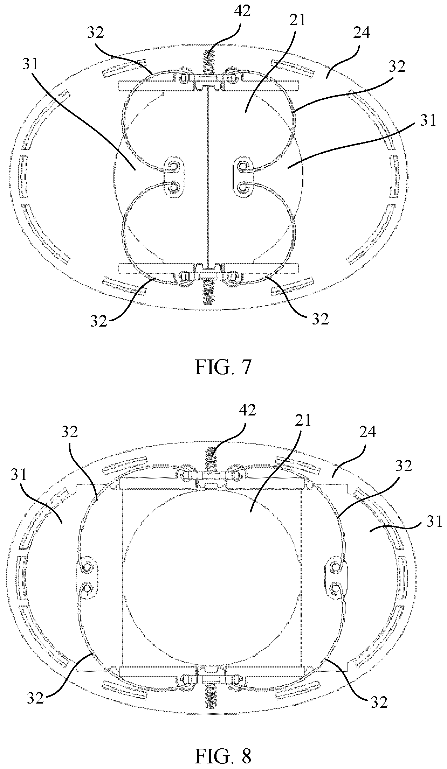

For the structure in which the door plate assembly 3 includes the two door plates 31, when each door plate 31 is connected to one first elastic member 32, although the door plate 31 can be opened, one first elastic member 32 has limited driving force, and it is difficult to ensure that the door plate 31 is evenly stressed in a movement direction. The door plate 31 shakes due to uneven stressing, and is prone to being stuck during a movement. Therefore, in this embodiment, as shown in FIG. 7 and FIG. 8, each door plate 31 is connected to the two first elastic members 32, so that each door plate 31 can obtain greater driving force, to improve a speed of opening the door plate 31. In addition, the two first elastic members 32 connected to each door plate 31 are symmetrically distributed, so that even driving force can be applied to the door plate 31. This ensures movement stability of the door plate 31 and facilitates quick opening of the door plate 31.

Specifically, as shown in FIG. 5 and FIG. 6, the door plate assembly 3 further includes a guide structure 33. The guide structure 33 is connected to the second housing 2. The door plate 31 is slidably connected to the guide structure 33. The door plate 31 is slidably connected to the guide structure 33, to ensure movement stability of the door plate 31, and facilitate quick opening of the door plate 31.

The guide structure 33 may be disposed on an inner side of the cover plate 24 of the second housing 2, so that the guide structure 33 can be shielded by the cover plate 24, to prevent contamination by an external dusty impurity and the like.

Specifically, the guide structure 33 may be a guide rail, and the guide rail may be fastened to the second housing 2 through a connector like a screw or a rivet. In this way, installation and fastening can be convenient.

In another embodiment, the guide structure 33 may alternatively be a slide groove formed on the second housing 2, and edge of the door plate 31 may be slidably disposed in the slide groove, or a guide protruding edge is formed on the second housing 2, and a slot that can be slidably connected to the guide protruding edge may be provided on the door plate 31.

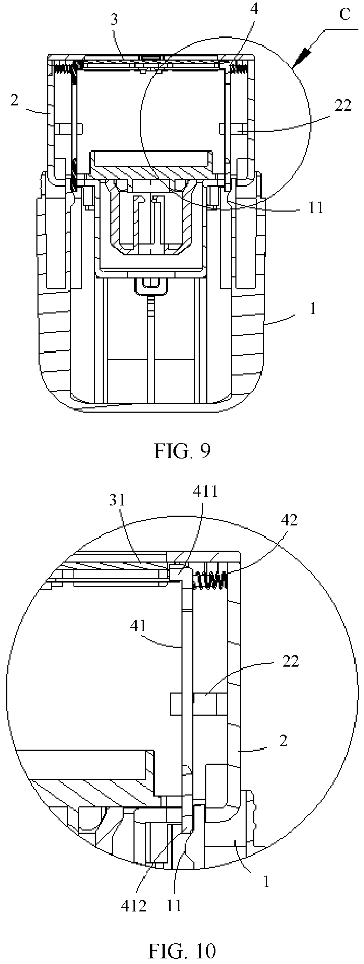

Specifically, as shown in FIG. 9, the implantation apparatus further includes limiting assemblies 4. The limiting assembly 4 is connected to the second housing 2, and is configured to limit the door plate assembly 3 to keep the sealed state of the through hole 21. When the door plate assembly 3 seals the through hole 21, the limiting assembly 4 can cooperate with the door plate assembly 3, to prevent the door plate assembly 3 from being opened. In this way, when the implantation apparatus is not used, the through hole 21 can be kept sealed through cooperation between the door plate assembly 3 and the limiting assembly 4.

Specifically, in an embodiment, as shown in FIG. 9 to FIG. 11, the limiting assembly 4 includes a lever 41 and a second elastic member. The lever 41 includes a first end 411 and a second end 412. Rotating parts 413 are disposed in positions that are of the lever 41 and that are located between the first end 411 and the second end 412. The rotating part 413 is rotatably connected to the second housing 2. One end of the second elastic member is connected to the second housing 2, and the other end of the second elastic member is connected to the first end 411 of the lever 41. An end that is of the first end 411 of the lever 41 and that is away from the second elastic member is configured to limit the door plate assembly 3 to keep the sealed state of the through hole 21. First protrusions 11 are disposed on inner side walls of the first housing 1. At least a part of the second end 412 of the lever 41 blocks the first protrusion 11.

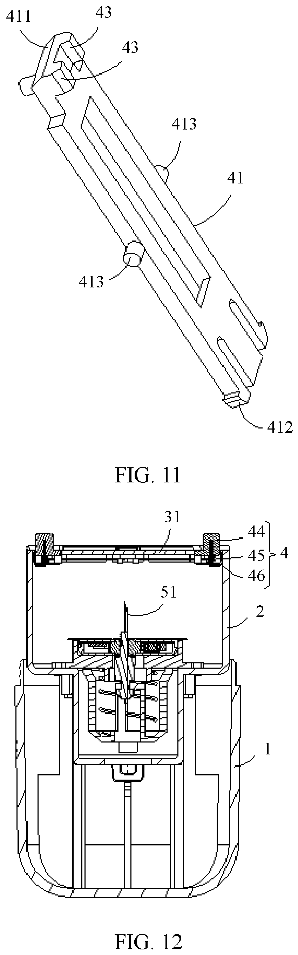

As shown in FIG. 11, the lever 41 is a rod-like structure, and two ends of the lever 41 are respectively formed as the first end 411 and the second end 412. Connection blocks 22 may be disposed on inner walls of the second housing 2, and the connection block 22 can protrude toward the inside of the second housing 2, to be rotatably connected to the lever 41. Specifically, the lever 41 may be rotatably connected to the connection block 22 through the rotating part 413 with a shaft-like structure.

As shown in FIG. 9 and FIG. 10, when the implantation apparatus is not used, the lever 41 may keep a vertical state. The first end 411 of the lever 41 may be in limiting cooperation with the door plate assembly 3 under acting force provided by the second elastic member, so that the door plate assembly 3 keeps the sealed state of the through hole 21. The second end 412 of the lever 41 may block and be connected to the first protrusion 11 in a vertical direction, that is, a projection of the second end 412 in the vertical direction at least partially coincides with the first protrusion 11. In this case, the second end 412 may or may not be in contact with the first protrusion 11, and there may be no interaction force between the second end 412 and the first protrusion 11. When the second housing 2 is pressed, the second housing 2 moves downward relative to the first housing 1, and the lever 41 moves downward synchronously with the second housing 2. Because the at least part of the second end 412 blocks the first protrusion 11, when passing through the first protrusion 11, the second end 412 is pushed toward the inside of the first housing 1 by the first protrusion 11, so that the rotating part 413 of the lever 41 rotates relative to the second housing 2, and the first end 411 of the lever 41 moves in a direction away from the door plate assembly 3. In this way, the first end 411 can be separated from the door plate assembly 3, and after the door plate assembly 3 is not constrained by the lever 41, the door plate 31 can be automatically opened under an action of the first elastic member 32. A surface that is of the first protrusion 11 and that faces the second housing 2 can be an oblique surface. In this way, when moving downward, the lever 41 can slide through guide of the oblique surface of the first protrusion 11 to pass through the first protrusion 11, to avoid sticking of the lever 41 at the first protrusion 11.

The second elastic member may be a spring, a dome, or the like, or certainly, may be elastic rubber, silicone, or the like.

Specifically, as shown in FIG. 11, a clamping jaw 43 is disposed at the first end 411 of the lever 41. A clamping slot 34 is provided on the door plate assembly 3. The clamping jaw 43 is in clamping cooperation with the clamping slot 34. When the door plate assembly 3 seals the through hole 21, the clamping jaw 43 of the lever 41 can be clamped into the clamping slot 34. This can prevent the door plate assembly 3 from being opened. When the second housing 2 is pressed down, the lever 41 can rotate to separate the clamping jaw 43 from the clamping slot 34, and the door plate assembly 3 that is not constrained by the clamping jaw 43 can be automatically opened.

When the door plate assembly 3 includes the two door plates 31, two clamping jaws 43 may exist. One clamping slot 34 may be provided on each door plate 31. The two clamping jaws 43 can be respectively in clamping cooperation with the clamping slots 34 of the two door plates 31, to limit the two door plates 31.

In another embodiment, as shown in FIG. 12 to FIG. 16, the limiting assembly 4 includes a button 44 and a third elastic member 45. The button 44 is slidably connected to the second housing 2. The button 44 partially protrudes from the upper surface of the second housing 2. A limiting part 441 is disposed on the button 44. The limiting part 441 is configured to limit the door plate assembly 3 to keep the sealed state of the through hole 21. One end of the third elastic member 45 is connected to the button 44, and the other end of the third elastic member 45 is connected to the second housing 2.

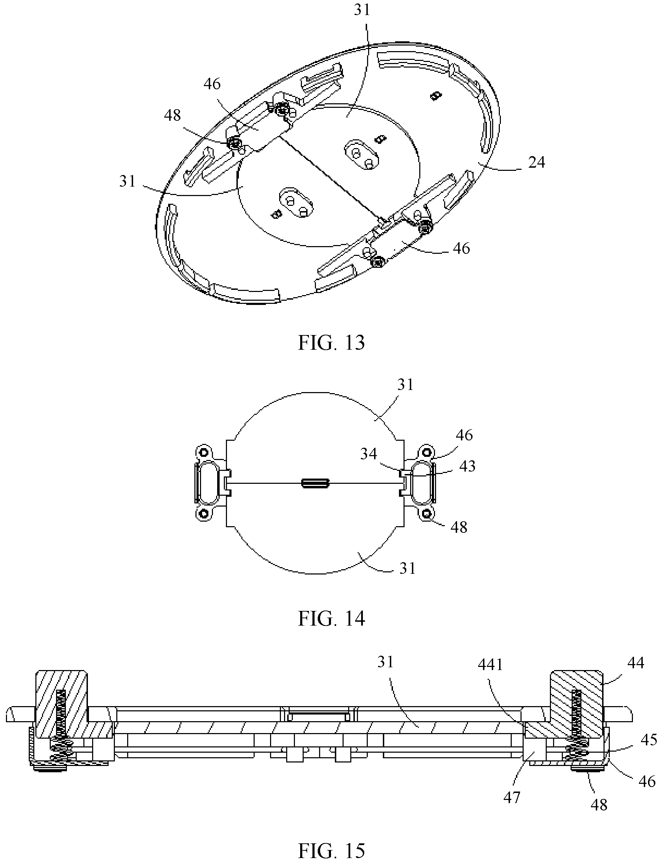

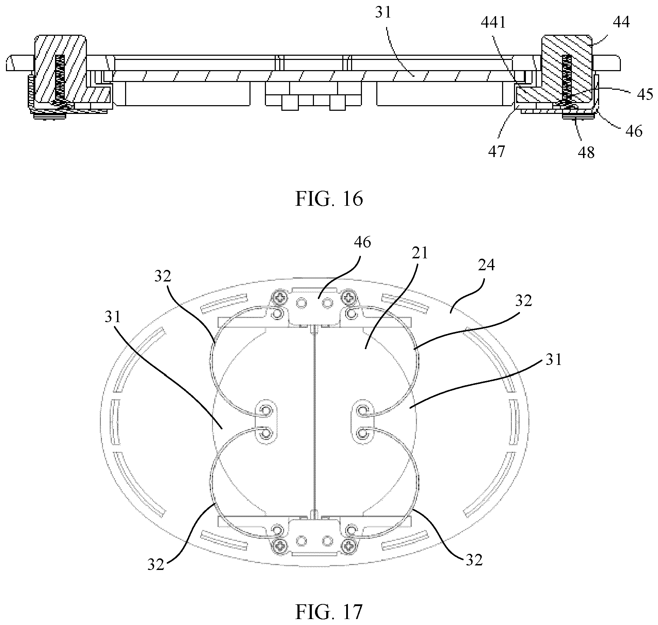

It may be understood that, to facilitate slide of the button 44 relative to the second housing 2, a hole may be provided on the second housing 2, the button 44 may slide in the hole, and at least a part of the button 44 can extend out from the hole, to facilitate pressing. Specifically, as shown in FIG. 15 and FIG. 16, when the user presses the second housing 2, because the button 44 protrudes from the upper surface of the second housing 2, the user preferably presses the button 44. The button 44 moves downward prior to the second housing 2, so that the limiting part 441 of the button 44 is separated from the door plate assembly 3. After the door plate assembly 3 is not constrained by the limiting part 441, the door plate assembly 3 can be automatically opened. When the door plate assembly 3 is not constrained by the limiting part 441, the skin of the user can be in contact with the upper surface of the second housing 2 and cover the through hole 21. In this way, in a process of opening the door plate assembly 3, because the through hole 21 is shielded by the skin, the user cannot see the internal implantation assembly through the through hole 21, reducing the psychological burden of the user.

The third elastic member 45 can support the button 44, so that when the implantation apparatus is not used, a partial position of the button 44 can protrude from the upper surface of the second housing 2. When the second housing 2 is pressed, the third elastic member 45 can be compressed, so that the button 44 can be pressed into the second housing 2. The third elastic member 45 may be a spring, a dome, or the like, or certainly, may be elastic rubber, silicone, or the like.

Specifically, as shown in FIG. 14, the limiting part 441 includes a clamping jaw 43. A clamping slot 34 is provided on the door plate assembly 3. The clamping jaw 43 is in clamping cooperation with the clamping slot 34. When the door plate assembly 3 seals the through hole 21, the clamping jaw 43 can be clamped into the clamping slot 34. This can prevent the door plate assembly 3 from being opened. When the button 44 is pressed down, the clamping jaw 43 can be separated from the clamping slot 34, and the door plate assembly 3 that is not constrained by the clamping jaw 43 can be automatically opened.

Specifically, as shown in FIG. 16 in FIG. 13, the limiting assembly 4 further includes a support member 46. The support member 46 is fastened to the second housing 2. Activity space 47 for the button 44 to move is disposed between the second housing 2 and a position that is of the support member 46 and that is opposite to the button 44. An end that is of the third elastic member 45 and that is away from the button 44 is connected to the support member 46.

The support member 46 may be a plastic member or a metal member, or certainly, may be another structure having specific hardness. The support member 46 can be fixedly connected to the second housing 2 through screws 48, to ensure reliable support for the button 44 and the third elastic member 45. The activity space 47 is disposed between the support member 46 and the second housing 2, so that the button 44 can move to the second housing 2 after being pressed. In this way, the limiting part 441 of the button 44 can be separated from the door plate assembly 3, a constraint on the door plate assembly 3 is canceled, and the door plate assembly 3 can be automatically opened.

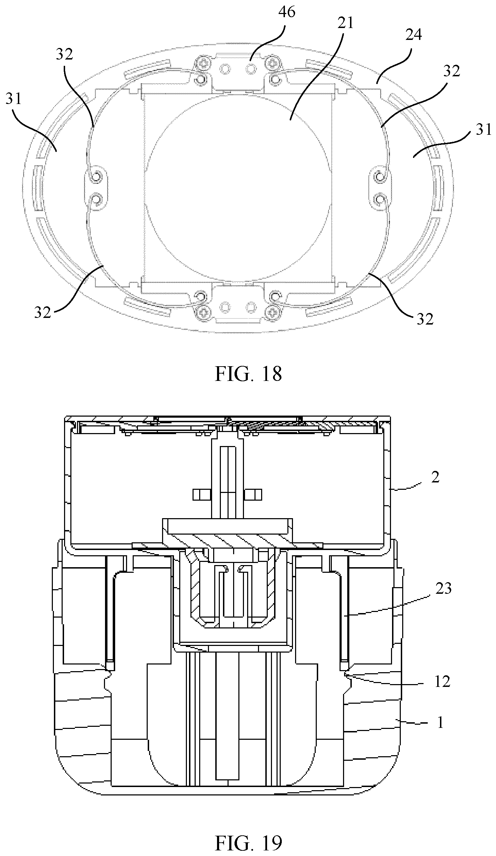

In this embodiment, through cooperation between the button 44, the third elastic member 45, and the support member 46, small space can be occupied, the door plate assembly 3 can be limited or unlimited, the structure is small and simple, and an operation is convenient. A status of the through hole 21 from being closed to being opened may alternatively be implemented through cooperation between the door plate 31 and the first elastic member 32, as shown in FIG. 17 and FIG. 18. Details are not described herein again.

Specifically, as shown in FIG. 19, support parts 23 are disposed at the bottom of the second housing 2. Second protrusions 12 are disposed on inner walls of the first housing 1. In a movement direction of the second housing 2, at least a part of the support part 23 laps the second protrusion 12.

When the implantation apparatus is not used, the second housing 2 can be supported on the second protrusion 12 of the first housing 1 via the support part 23, to prevent the second housing 2 from falling into the first housing 1. When the second housing 2 is pressed under appropriate external force, the support part 23 can undergo appropriate elastic deformation after being stressed. In one aspect, the support part can absorb the force applied to the second housing 2, to buffer a movement of the second housing 2, so as to prevent bringing discomfort to the user when the second housing 2 falls down quickly when being greatly stressed. In another aspect, the support part can deviate through elastic deformation, to cancel a lapping state with the second protrusion 12, so that the support part 23 can pass through the second protrusion 12 with the movement of the second housing 2, the second housing 2 can smoothly fall into the first housing 1, and the implantation assembly can complete an implantation action after extending out from the through hole 21.

The foregoing descriptions are merely preferred embodiments of this application, and are not intended to limit this application. For a person skilled in the art, various changes and variations may be made to this application. Any modification, equivalent replacement, improvement, and the like made within the spirit and principle of this application shall fall within the protection scope of this application.

## Claims

1. An implantation apparatus, comprising:
a first housing, wherein an implantation assembly is disposed in the first housing;
a second housing, wherein the second housing movably penetrates the first housing, and a through hole for the implantation assembly to pass through is provided on the first housing; and
a door plate assembly, movably connected to the second housing, and configured to seal or open the through hole.

2. The implantation apparatus according to claim 1, wherein the door plate assembly comprises a door plate and a first elastic member, one end of the first elastic member is connected to the second housing, the other end of the first elastic member is connected to the door plate, and the first elastic member is in a compressed state when the door plate is in a state of sealing the through hole.

3. The implantation apparatus according to claim 2, wherein two door plates are symmetrically disposed, each door plate is connected to the second housing through at least one first elastic member, and the two door plates move reversely through the first elastic members respectively.

4. The implantation apparatus according to claim 3, wherein each door plate is connected to the second housing through two first elastic members, and in a movement direction perpendicular to the door plate, two first elastic members connected to one door plate are symmetrically distributed.

5. The implantation apparatus according to any one of claims 2 to 4, wherein the door plate assembly further comprises a guide structure, the guide structure is connected to the second housing, and the door plate is slidably connected to the guide structure.

6. The implantation apparatus according to claim 5, wherein the guide structure is a guide rail.

7. The implantation apparatus according to any one of claims 1 to 4, wherein the implantation apparatus further comprises a limiting assembly, and the limiting assembly is connected to the second housing, and is configured to limit the door plate assembly to keep a sealed state of the through hole.

8. The implantation apparatus according to claim 7, wherein the limiting assembly comprises a lever and a second elastic member, the lever comprises a first end and a second end, a rotating part is disposed in a position that is on the lever and that is located between the first end and the second end, and the rotating part is rotatably connected to the second housing;
one end of the second elastic member is connected to the second housing, the other end of the second elastic member is connected to the first end of the lever, and an end that is of the first end of the lever and that is away from the second elastic member is configured to limit the door plate assembly to keep the sealed state of the through hole; and
a first protrusion is disposed on an inner side wall of the first housing, and at least a part of the second end of the lever blocks the first protrusion.

9. The implantation apparatus according to claim 8, wherein a clamping jaw is disposed at the first end of the lever, a clamping slot is provided on the door plate assembly, and the clamping jaw is in clamping cooperation with the clamping slot.

10. The implantation apparatus according to claim 7, wherein the limiting assembly comprises a button and a third elastic member, the button is slidably connected to the second housing, the button partially protrudes from an upper surface of the second housing, a limiting part is disposed on the button, and the limiting part is configured to limit the door plate assembly to keep the sealed state of the through hole; and
one end of the third elastic member is connected to the button, and the other end of the third elastic member is connected to the second housing.

11. The implantation apparatus according to claim 10, wherein the limiting part comprises a clamping jaw, a clamping slot is provided on the door plate assembly, and the clamping jaw is in clamping cooperation with the clamping slot.

12. The implantation apparatus according to claim 10, wherein the limiting assembly further comprises a support member, the support member is fastened to the second housing, activity space for the button to move is disposed between the second housing and a position that is of the support member and that is opposite to the button, and an end that is of the third elastic member and that is away from the button is connected to the support member.

13. The implantation apparatus according to any one of claims 1 to 12, wherein a support part is disposed at a bottom of the second housing, a second protrusion is disposed on an inner wall of the first housing, and in a movement direction of the second housing, at least a part of the support part laps the second protrusion.
